# EUROPEAN PATENT APPLICATION

(11) **EP 4 711 379 A1**
(43) Date of publication of application: **18.03.2026**
(21) Application number: 25156613.9
(22) Date of filing: 07.02.2025
(51) Int. Cl.: C07K 14/33, C12N 9/52, C12N 15/70

(54) **METHOD FOR PRODUCING BOTULINUM NEUROTOXIN USING SOLUBLE PARTNER AND GP41.1 INTEIN**

(30) Priority: 07.03.2024 KR 20240032820
(71) Applicant: Mvrix Co., Ltd., Gyeonggi-do 18469 (KR)
(72) Inventor: Jung, Sang won, 05507 Seoul (KR); Kweon, Dae Hyuk, 04129 Seoul (KR); Lee, Chi Ho, 13908 Gyeonggi-do (KR); Kim, Byoung hee, 15823 Gyeonggi-do (KR); Park, Song, 36719 Gyeongsangbuk-do (KR)
(74) Representative: RGTH

(57) **Abstract**

Disclosed is a method of producing botulinum toxin by a recombinant method using a soluble partner and GP41.1 intein. It was found that a heavy chain receptor binding domain (HC) of botulinum toxin with low solubility may be expressed in a soluble form in *E. coli* by linking a soluble partner. In addition, it was found that the soluble partner, which is generally a protein having a very large molecular weight, is easily removed using GP41.1 intein.

## Description

### BACKGROUND OF THE INVENTION

### Field of the Invention

The present invention relates to a method of producing botulinum neurotoxin using a soluble partner and GP41.1 intein.

### Description of the Related Art

Botulinum toxin is a neurotoxic protein produced by the bacteria *Clostridium botulinum* strain, which specifically acts on SNAREs present in nerve cells, inhibits complex formation, and inhibits membrane fusion, thereby blocking the release of neurotransmitters. This causes inhibition of muscle movement or the sympathetic or parasympathetic nervous system. Based on these effects, botulinum toxin is known to be effective in treating various diseases.

Specifically, botulinum toxin is known to be mainly used for cosmetic procedures such as wrinkle removal and is also used to treat many diseases associated with neurotransmitter secretion or muscles such as strabismus, blepharospasm, vocal cord disorders, torticollis, myocardial disorders, ulcers and gastroesophageal reflux disease, decreased appetite, pancreatic diseases, stretch marks, urgency incontinence, fissures, poliomyelitis, myalgia, hip deformities, hyperhidrosis, back pain, cervical pain, chronic headaches, and cranial nerve disorders.

Botulinum neurotoxin (BoNT) is largely divided into two parts: a heavy chain (HC, approximately 100 kDa) and a light chain (LC, approximately 50 kDa) which has enzymatic activity that cleaves SNARE complex-forming proteins in nerve cells. The heavy chain (HC) is further divided into a part (receptor-binding domain, RBD or H_{C}) that recognizes and binds to nerve cells and a part (translocation domain, H_{N}) that translocates the light chain to the nerve cytoplasm.

Meanwhile, after botulinum toxin is released from bacteria in the form of a single chain, it is cleaved into two chains, namely, the heavy chain (HC) and the light chain (LC), by an endogenous protease, and the two chains are linked to each other again through a disulfide bond, and with a resultant size of approximately 150 kDa. Therefore, single-chain botulinum toxin released from bacteria must be cleaved and linked again, which inevitably reduces yield and increases production costs. In addition, botulinum toxin is highly hazardous, thus entailing a high cost for production license and the resulting safety equipment.

In addition, screening for strains producing botulinum toxin in nature is a very difficult process. Strains producing the desired type of toxin should be found and these strains should have sufficient productivity. It is also very difficult to produce full-length toxin in recombinant *E. coli,* not *Clostridium botulinum.* In particular, it is difficult to express botulinum toxin having a size of 150 kDa in a soluble form in *E. coli* cells. In addition, botulinum toxin has interchain and intrachain disulfide bonds, whereas *E. coli* cells have a problem of having not disulfide bonds. In addition, as described above, botulinum toxin should have a structure in which the peptide bond between the light chain and the heavy chain is accurately cleaved and the chains are linked again by a disulfide bond. On the other hand, *E. coli* does not contain a protein for cleaving and linking disulfide bonds as described above and thus has a problem of requiring an additional separate process.

### [Prior art literature]

### [Patent literature]

(Patent literature 1) Korean Patent No. 10-2610179 (2023. 11. 30) discloses a method of producing botulinum toxin using *E. coli.*
(Patent literature 2) Korean Patent Publication Laid-open No. 10-2020-0115584 (2020.10. 07) discloses a method of producing botulinum toxin using *Bacillus* as a host.

### SUMMARY OF THE INVENTION

Therefore, the present invention has been made in view of the above problems, and it is one object of the present invention to provide a method of safely and easily producing botulinum toxin through genetic recombination.

It is another object of the present invention to provide a method of producing botulinum toxin in a high yield by expressing the botulinum toxin in a soluble form.

In accordance with an aspect of the present invention, the above and other objects can be accomplished by the provision of a method of producing botulinum toxin including 1) expressing and producing each of a botulinum toxin fragment, "LC-H_{N}-GP41.1^{N}", including a light chain (LC) of botulinum toxin, a heavy chain translocation domain (H_{N}) of botulinum toxin, and GP41.1 intein^{N} (GP41.1^{N}) sequentially linked from a 5' end to a 3' end, and a botulinum toxin fragment, "SP-GP41.1^{C}-H_{C}", including a soluble partner (SP) containing polypeptide, GP41.1 intein C (GP41.1^{C}), and a heavy-chain receptor-binding domain (H_{C}) of botulinum toxin sequentially linked from the 5' end to the 3' end, 2) purifying each of the LC-H_{N}-GP41.1^{N} and the SP-GP41.1^{C}-H_{C}, 3) removing "GP41.1^{N}" linked to the heavy chain translocation domain (H_{N}) and "SP-GP41.1^{C}" linked to the heavy-chain receptor-binding domain (H_{C}) using a protein trans-splicing method and linking the heavy chain translocation domain (H_{N}) to the heavy-chain receptor-binding domain (H_{C}) without the soluble partner (SP), and 4) cleaving the peptide bond between the light chain (LC) and the heavy chain translocation domain (H_{N}), and inducing a disulfide bond between the cleaved light chain (LC) and heavy chain translocation domain (H_{N}).

In accordance with another aspect of the present invention, there is provided a method of producing botulinum toxin including 1) expressing and producing each of a botulinum toxin fragment, "LC-H_{N}-GP41.1^{N}", including a light chain (LC) of botulinum toxin, a heavy chain translocation domain (H_{N}) of botulinum toxin, and GP41.1 intein^{N} (GP41.1^{N}) sequentially linked from a 5' end to a 3' end, and a botulinum toxin fragment, "SP-GP41.1^{C}-H_{C}", including a soluble partner (SP) containing polypeptide, GP41.1 intein C(GP41.1^{C}), and a heavy-chain receptor-binding domain (H_{C}) of botulinum toxin sequentially linked from the 5' end to the 3' end, 2) purifying each of the LC-H_{N}-GP41.1^{N} and the SP-GP41.1^{C}-H_{C}, 3) cleaving the peptide bond between the light chain (LC) and the heavy chain translocation domain (H_{N}), and inducing a disulfide bond between the cleaved light chain (LC) and heavy chain translocation domain (H_{N}), and 4) removing "GP41.1^{N}" linked to the heavy chain translocation domain (H_{N}) and "SP-GP41.1^{C}" linked to the heavy-chain receptor-binding domain (H_{C}) using a protein trans-splicing method and linking the heavy chain translocation domain (H_{N}) to the heavy-chain receptor-binding domain (H_{C}) without the soluble partner (SP).

The GP41.1 intein N (GP41.1^{N}) and the soluble partner may be further bound with a purification tag.

The expression may occur in *E. coli.*

### BRIEF DESCRIPTION OF THE DRAWINGS

The above and other objects, features and other advantages of the present invention will be more clearly understood from the following detailed description taken in conjunction with the accompanying drawings, in which:

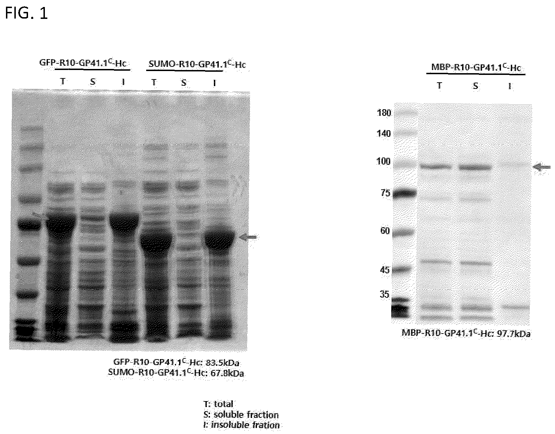

FIG. 1 shows the result of determination as to whether or not a botulinum toxin fragment, soluble partner-R10-GP41.1^{C}-H_{C}, is expressed in a soluble form in *E. coli* using various types of soluble partners (GFP, SUMO, MBP);

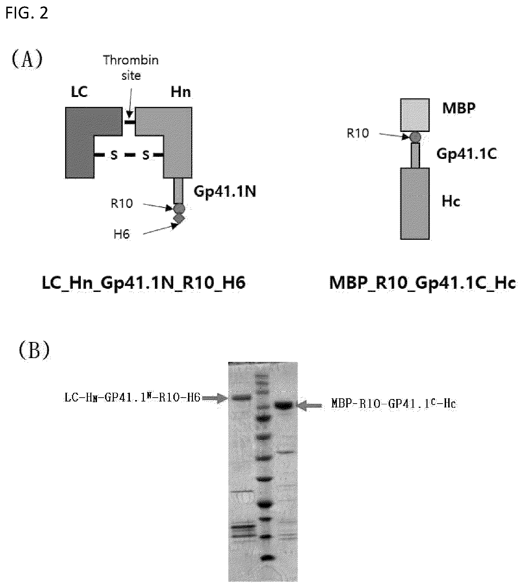

FIG. 2(A) schematically shows the structures of the botulinum toxin fragment MBP-R10-GP41.1^{C}-H_{C} and the botulinum toxin fragment LC-H_{N}-GP41.1^{N}-R10-H6, and FIG. 2(B) shows the results of identification using SDS-PAGE of the botulinum toxin fragment MBP-R10-GP41.1^{C}-H_{C} and the botulinum toxin fragment LC-H_{N}-GP41.1^{N}-R10-H6 in *E. coli;*

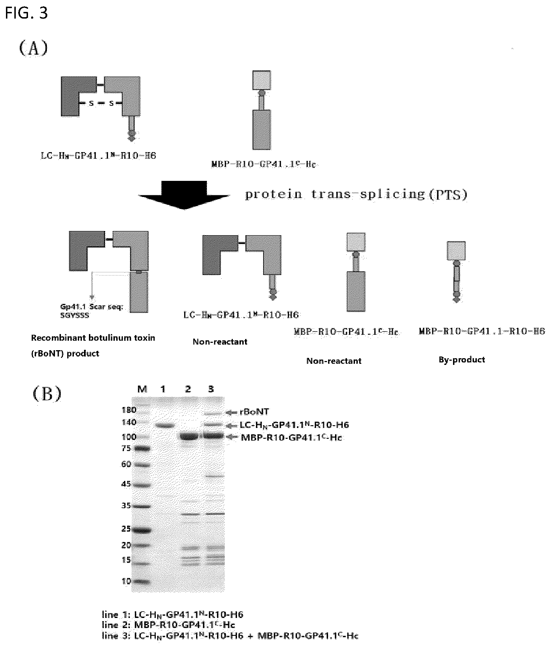

FIG. 3(A) shows a process of mixing botulinum toxin fragment MBP-R10-GP41.1^{C}-H_{C} with the botulinum toxin fragment LC-H_{N}-GP41.1^{N}-R10-H6 to induce protein trans splicing and thereby produce a recombinant botulinum toxin (LC-H_{N}, rBoNT) and FIG. 3(B) shows the result of identification using SDS-PAGE of the protein trans splicing product.

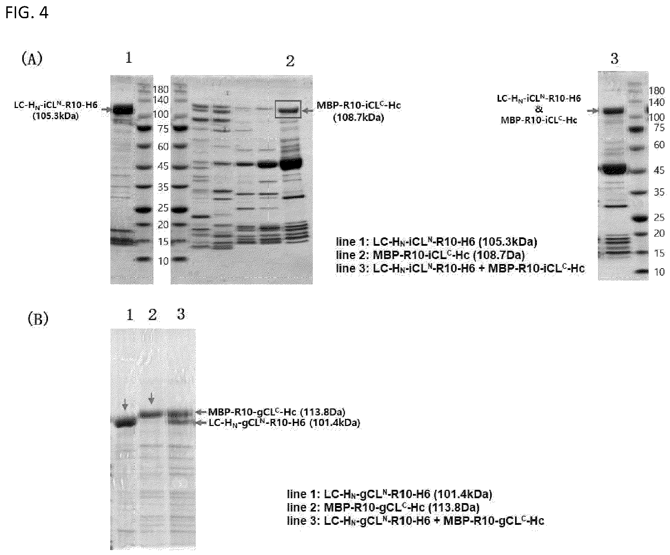

FIG. 4(A) shows the result of identification using SDS-PAGE of the result of protein trans splicing induced by mixing MBP-R10-iCL^{C}-H_{C} with the botulinum toxin fragment LC-H_{N}-iCL^{N}-R10-H6 and FIG. 4(B) shows the result of identification using SDS-PAGE of the result of protein trans splicing induced by mixing MBP-R10-iCL^{C}-H_{C} with the botulinum toxin fragment LC-H_{N}-iCL^{N}-R10-H6;

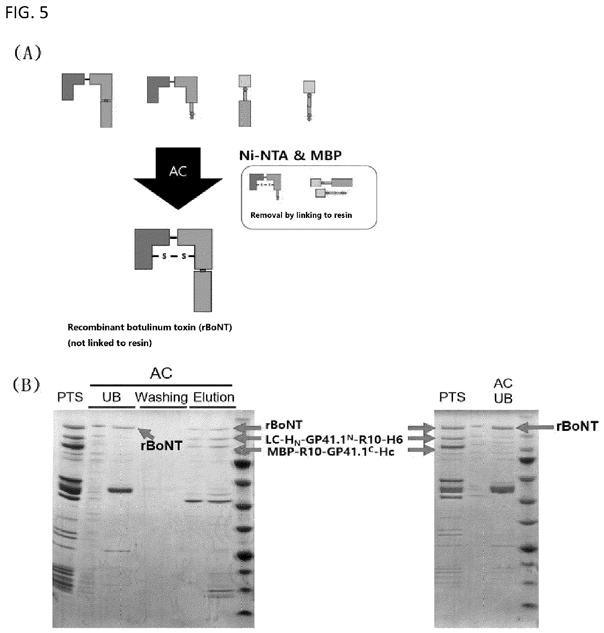

FIG. 5(A) schematically illustrates a process of removing reaction by-products (MBP-R10-GP41.1-R10-H6) and non-reactants (LC-H_{N}-GP41.1^{N}-R10-H6, MBP-R10-GP41.1^{C}-H_{C}) by performing affinity chromatography (AC) on the protein trans-splicing reaction product (PTS) and FIG. 5(B) shows the results of identification using SDS-PAGE of an unbinding (UB) fraction, an elution fraction, and a washing fraction obtained through affinity chromatography; and

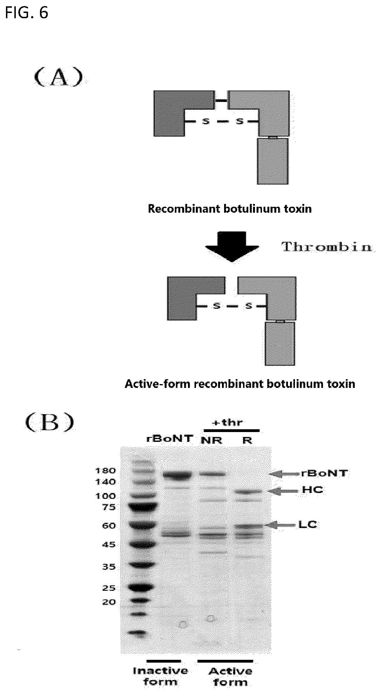

FIG. 6(A) schematically illustrates a process of cleaving the light chain (LC) of botulinum toxin and the heavy chain botulinum toxin (H_{N}) by treating the recombinant botulinum toxin (LC-H_{N}, rBoNT) with thrombin (thr) and FIG. 6(B) shows the results of comparison between a sample of thrombin-treated recombinant botulinum toxin treated with a reducing agent (reduced, R) and a sample not treated with a reducing agent (non-reduced, NR) using SDS-PAGE to determine whether or not the recombinant botulinum toxin is accurately cleaved by thrombin treatment.

### DETAILED DESCRIPTION OF THE INVENTION

The present invention provides a method of producing botulinum toxin including: 1) expressing and producing each of a botulinum toxin fragment, "LC-H_{N}-GP41.1^{N}", including a light chain (LC) of botulinum toxin, a heavy chain translocation domain (H_{N}) of botulinum toxin, and GP41.1 intein^{N} (GP41.1^{N}) sequentially linked from a 5' end to a 3' end, and a botulinum toxin fragment, "SP-GP41.1^{C}-H_{C}", including a soluble partner (SP) containing polypeptide, GP41.1 intein C (GP41.1^{C}), and a heavy-chain receptor-binding domain (H_{C}) of botulinum toxin sequentially linked from the 5' end to the 3' end; 2) purifying each of the LC-H_{N}-GP41.1^{N} and the SP-GP41.1^{C}-H_{C}; 3) removing "GP41.1^{N}" linked to the heavy chain translocation domain (H_{N}) and "SP-GP41.1^{C}" linked to the heavy-chain receptor-binding domain (H_{C}) using a protein trans-splicing method and linking the heavy chain translocation domain (H_{N}) to the heavy-chain receptor-binding domain (H_{C}) without the soluble partner (SP); and 4) cleaving the peptide bond between the light chain (LC) and the heavy chain translocation domain (H_{N}), and inducing a disulfide bond between the cleaved light chain (LC) and heavy chain translocation domain (H_{N}).

The present inventors developed a method of safely producing botulinum toxin by producing each of a botulinum toxin fragment including a light chain (LC) of botulinum toxin and a heavy chain translocation domain (H_{N}) of botulinum toxin, and a botulinum toxin fragment including a heavy-chain receptor-binding domain (H_{C}) of botulinum toxin, and then linking the botulinum toxin fragments to each other using a Cfa intein.

However, the method of producing botulinum toxin as described above has a problem in that the botulinum toxin fragment including the heavy-chain receptor-binding domain (H_{C}) of botulinum toxin is often expressed in an insoluble form in *E. coli,* resulting in a low yield upon production.

In addition, when botulinum toxin fragments are linked using the cfa intein, residues containing cysteine are left. The cysteine residue may cause a problem that the produced recombinant botulinum toxin forms a disulfide bond and creates a dimer. In addition, in order for the botulinum toxin to be completely active, a disulfide bond mediated by cysteine should be formed between LC and H_{N}. Cysteine-containing scars may cause a problem in which a disulfide bond which is different from the original disulfide bond may be formed, which may decrease the activity of the botulinum toxin.

However, the present invention is based on the finding that, when GP41.1, among various types of inteins that do not leave cysteine residues, is used, the botulinum toxin fragments can be linked while the purification tag or soluble partner linked to GP41.1^{N} or GP41.1^{C} is removed.

In addition, the present invention is based on the finding that maltose-binding protein (MBP), among various soluble partners, is particularly highly effective in expressing the protein linked to the heavy-chain receptor-binding domain (H_{C}) of botulinum toxin and GP41.1 in a soluble form.

Hereinafter, each step of the method of producing the botulinum toxin of the present invention will be described in more detail.

### <Step 1: Production of botulinum toxin fragments>

In this step, each of a botulinum toxin fragment, "LC-H_{N}-GP41.1^{N}", including a light chain (LC) of botulinum toxin, a heavy chain translocation domain (H_{N}) of botulinum toxin, and GP41.1 intein^{N} (GP41.1^{N}) sequentially linked from a 5' end to a 3' end, and a botulinum toxin fragment, "SP-GP41.1^{C}-H_{C}", including a soluble partner (SP) containing polypeptide, GP41.1 intein C (GP41.1^{C}), and a heavy-chain receptor-binding domain (H_{C}) of botulinum toxin sequentially linked from the 5' end to the 3' end are expressed and produced. This step is characterized in that the botulinum toxin fragments are expressed in soluble forms.

In general, methods of producing botulinum toxin by expressing the entire sequence of botulinum toxin or by separately expressing the light chain (LC) of botulinum toxin and the heavy chain (HC) of botulinum toxin are known.

However, this step is characterized in that botulinum toxin with higher solubility is expressed by separately expressing a botulinum toxin fragment including a light chain (LC) of botulinum toxin and a heavy chain translocation domain (H_{N}) of botulinum toxin, and a botulinum toxin fragment including a heavy-chain receptor-binding domain (H_{C}) of botulinum toxin.

In addition, this step is characterized in that a botulinum toxin fragment including the heavy-chain receptor-binding domain (H_{C}) of botulinum toxin is expressed with higher solubility by further linking a soluble partner to the heavy-chain receptor-binding domain (H_{C}) of botulinum toxin. It can be seen from the following examples of the present invention that, using a soluble partner, the solubility expression of the heavy-chain receptor-binding domain (H_{C}) of botulinum toxin can be increased and botulinum toxin can be produced at a higher yield.

In this step, the soluble partner refers to a partner that increases a soluble expression when linked to the botulinum toxin fragment and examples thereof include GFP, SUMO, and MBP. Meanwhile, it can be seen from the following examples that, among the soluble partners, maltose-binding protein (MBP) has a particularly excellent solubility expression effect on the botulinum toxin fragment (GP41.1^{C}-H_{C}) including GP41.1 intein C and the heavy-chain receptor-binding domain (H_{C}) of botulinum toxin, which are linked to each other.

Meanwhile, when the soluble partner is used, the soluble partner should be removed separately in order to obtain a botulinum toxin product. However, there is a problem in that the soluble partner is generally a protein having a very large molecular weight and thus additional genetic manipulation such as insertion of a restriction enzyme recognition sequence is required in order to remove the soluble partner. On the other hand, it can be seen from the following Example that intein is used in the present invention and is easily removed without separate genetic manipulation.

Meanwhile, in the present invention, the intein is a self-splicing protein that contains intein N (N-terminal intein) and intein C (C-terminal intein). Respective inteins are separated from each other in the cells and are folded after they contact each other. These inteins act as an enzyme, conjugate the proteins (so-called exteins) located at the side end of respective inteins to each other, and self-eliminate. In the present invention, when, among various types of inteins that do not leave cysteine residues, GP41.1 is used, the botulinum toxin fragments can be linked by removing the purification tag and soluble partner linked to GP41.1^{N} or GP41.1^{C}. The 3'-end of intein N and the 5'-end of intein C may further include a linker sequence.

Meanwhile, in this step, the botulinum toxin fragment, "LC-H_{N}-Intein N", preferably further includes a purification tag at the 3'-end. As a result, the purification can be more smoothly performed in the subsequent purification process. In this case, the purification tag is not limited thereto and may be selected from a histidine tag, a lysine tag, and maltose-binding protein (MBP).

Meanwhile, expression at this step may be achieved using a plasmid in various host cells, such as *E. coli.*

### <Step 2: Purification of botulinum toxin fragments>

This step is a process of each purifying the botulinum toxin fragment LC-H_{N}-GP41.1^{N} and botulinum toxin fragment SP-GP41.1^{C}-H_{C} produced in step 1.

The purification method in this step may be performed by further linking a purification tag to the botulinum toxin fragment, expressing the same, and simply purifying the same using the purification tag, but is not limited thereto. Meanwhile, in the present invention, a maltose-binding protein was used as the purification tag due to the affinity to bind to maltose.

### <Step 3: Binding of botulinum toxin fragments using protein trans-splicing>

This step is a process of simultaneously removing "GP41.1^{N}" linked to the heavy chain translocation domain (H_{N}) and "SP-GP41.1^{C}" linked to the heavy-chain receptor-binding domain (H_{C}) using a protein trans-splicing method using GP41.1 intein, and linking the heavy chain translocation domain (H_{N}) and the heavy-chain receptor-binding domain (H_{C}) without the soluble partner (SP), to produce a full-length botulinum toxin (LC-H_{N}-H_{C}).

Meanwhile, when intein N and intein C contact, they are linked to each other to form a complete intein complex. When the complete intein complex is spliced, the extein proteins on the outside of the intein complex are linked by peptide bonds and become a single protein.

This step is a process of inducing a protein trans-splicing reaction using the inteins. In the present invention, it was confirmed that the protein trans-splicing reaction occurs completely although the purification tag and the soluble partner are linked to the inside of GP41.1 intein N (GP41.1^{N}) and GP41.1 intein C (GP41.1^{C}), and the purification tag linked to the C-terminus of intein N and the soluble partner linked to the N-terminus of intein C can be removed together when the intein complex is removed.

That is, the process whereby intein N and intein C recognize each other and are structurally linked should first be performed in order to perform protein trans-splicing using split inteins. For this purpose, the protein tag located in front or behind the inteins must not interfere with the interaction therebetween and the protein tag must not interfere with functional joining thereafter. On the other hand, GP41.1 intein according to the present invention has a configuration in which a botulinum toxin fragment is present on the outside and a purification tag or a soluble tag is present on the inside, which may interfere with binding and function, but the binding force is not interfered with by the interaction between intein N and intein C, and steric hindrance does not occur, whereby the protein trans-splicing process can be performed completely.

Meanwhile, the feature of this step in which the purification tag and soluble partner are removed together when the intein complex is removed, as described above, provides an advantage of eliminating the necessity to separately perform a process for removing other proteins such as the purification tag and soluble partner linked to the botulinum toxin through peptide bonds.

In addition, this step has another advantage of simplifying the process of purifying the botulinum toxin after the protein trans-splicing reaction. Specifically, when the botulinum toxin fragments 'LC-H_{N}-GP41.1^{N}-H6' and 'H6-SP-GP41.1^{C}-H_{C}', which are produced further using a purification tag such as a histidine tag, are mixed to induce a protein trans-splicing reaction, impurities such as non-reactants (LC-H_{N}-Intein N-H6, H6-SP-Intein C-H_{C}) and by-products (H6-Intein-SP) as well as the full-length botulinum toxin (BoNT) may be mixed together (see A of FIG. 3). The impurities contain the histidine tag (H6), which is a purification tag and thus can be easily removed through affinity chromatography using the histidine tag. Meanwhile, when the soluble partner (SP) acts as a purification tag, the effect can be obtained without using a separate purification tag, such as SP-Intein C-H_{C} (see A of FIG. 5).

Meanwhile, in this step, when the intein complex is removed through the protein trans-splicing reaction, a partial residual sequence (so-called "scar") of the intein complex may remain. Therefore, in the present invention, it is preferable to use the type of intein that does not leave a cysteine residue and more preferably, to use GP41.1. GP41.1 leaves SGYSSS as a residual sequence in the protein trans-splicing process. GP41.1 provides an advantage of not forming unnecessary disulfide bonds because the residual sequence contains no cysteine (C). In addition, in the present invention, it was found that, when among various types of inteins that do not leave a cysteine residue, GP41.1 is used, a botulinum toxin fragment can be linked while removing the purification tag and soluble partner linked to GP41.1^{N} or GP41.1^{C}.

### <Step 4: Cleavage of peptide bond between botulinum toxin light chain (LC) and heavy chain translocation domain (H_{N}) and induction of disulfide bond>

In order for botulinum toxin to exhibit full activity, the light chain (LC) and the heavy chain translocation domain (H_{N}) must be linked by a disulfide bond, not a peptide bond. This step is a process of cleaving the peptide bond between the light chain (LC) and the heavy- chain translocation domain (H_{N}) of the full-length botulinum toxin (LC-H_{N}-H_{C}) obtained through step 3 and inducing a disulfide bond between the cleaved light chain (L_{C}) and the heavy chain translocation domain (H_{N}), whereby a botulinum toxin having full activity can be produced.

Meanwhile, this step may be performed before performing step 3.

Meanwhile, the process of cleaving the peptide bond between the light chain (LC) of the botulinum toxin and the heavy chain translocation domain (H_{N}) in this step may be performed by protease treatment. That is, when a protease recognition sequence is inserted between LC and H_{N}, and the botulinum toxin fragment is expressed during production of the botulinum toxin fragment, LC-H_{N}-GP41.1^{N}, in step 1, the peptide bond between LC and H_{N} can be easily cleaved by treatment with a protease in this step.

Meanwhile, the formation of the disulfide bond between the light chain (LC) of the botulinum toxin and the heavy chain translocation domain (H_{N}) in this step may be induced by treatment with an oxidizing agent. The disulfide bond may be already formed when the botulinum toxin fragment, LC-H_{N}-Intein N, is expressed in step 1, a separate treatment process may not be necessary. That is, the light chain (LC) and the heavy chain translocation domain (HN) of botulinum toxin contain cysteine and thus a disulfide bond is naturally formed when expressed in *E. coli.* When there is no separate treatment with a reducing agent, the disulfide bond may be maintained intact and thus separate treatment may not be necessary.

Hereinafter, the present invention will be described in more detail with reference to the following examples, but the scope of the present invention is not limited to the examples, and includes variations and technical concepts equivalent thereto.

### [Example 1: Screening for soluble partner suitable for botulinum toxin production method of present invention]

Producing the heavy chain receptor-binding domain (HC) of the botulinum toxin in *E. coli* may cause a problem in that most of botulinum toxin is expressed insoluble.

In this example, whether or not soluble expression is increased when a soluble partner is linked to the heavy- chain receptor-binding domain (H_{C}) of botulinum toxin linked with intein C (intein C) was determined. In addition, a soluble partner having the best effect among the various soluble partners (GFP, SUMO, MBP) was determined.

A plasmid (SP-R10-GP41.1^{C}-H_{C}) was constructed by sequentially linking genes encoding soluble partners (GFP, green fluorescent protein, SEQ ID NO: 1; SUMO, a small ubiquitin-like modifier, SEQ ID NO: 2; MBP, maltose-binding protein, SEQ ID NO: 3), a linker (R10, SEQ ID NO: 4), GP41.1^{C} Intein C (SEQ ID NO: 5), and a heavy-chain receptor-binding domain of botulinum toxin (H_{C}, SEQ ID NO: 13) and then transformed into *E. coli* BL21 (DE3).

The transformed *E. coli* was inoculated into 10 ml of terrific broth and seed-cultured at 37°C and 200 rpm for 16 hours. 8 g of glycerol was added to 1 L of terrific broth to prepare a main culture medium, 10 ml of the seed-cultured solution was inoculated into the culture medium and main culture was performed at 37°C and 200 rpm for 2 hours until the OD₆₀₀ of the culture reached 0.5. IPTG was added to the main cultured solution at a concentration of 1 mM and main culture was performed at 16°C and 200 rpm for 16 hours.

The main culture solution was centrifuged at 4°C and 5,210 × g for 15 minutes and the supernatant was removed. The pellet obtained by removing the supernatant was resuspended in 150 ml of buffer (50 mM HEPES, 150 mM NaCl, pH 7.4) and the cells were disrupted using an ultrasonic disruptor (10 kHz, 1 sec on/2 sec off, 30 min). 1 ml of the disrupted cells were centrifuged at 19,800 x g and the supernatant was separated from the pellet.

Then, SDS-PAGE was performed using the supernatant obtained through the process as a soluble fraction (S) and using the pellet as an insoluble fraction (I) (FIG. 1).

As can be seen from FIG. 1, when GFP or SUMO was used as the soluble partner, a larger band appeared in the insoluble fraction (I). On the other hand, when MBP was used as the soluble partner, a larger band appeared in the soluble fraction (S).

In general, the heavy chain receptor-binding domain (H_{C}) of botulinum toxin has very low solubility. It can be seen from this example that heavy chain receptor-binding domain (H_{C}) of botulinum toxin was expressed in an increased solubility through soluble partners such as GFP, SUMO, and MSP, and that the effect of enhancing expression of solubility of "GP41.1^{C}-H_{C}" was particularly excellent when MBP was used.

### [Example 2: Production of botulinum toxin using soluble partner and intein]

The method of producing botulinum toxin according to the present invention is characterized in that botulinum toxin is produced by producing two botulinum toxin fragments of LC-H_{N}-intein N and SP-intein C-H_{C}, and then conjugating H_{N} with H_{C} using a protein trans-splicing method.

In this example, when the protein trans-splicing is performed as described above, whether or not the soluble partner (SP) linked to the N-terminus of intein C interferes with the action of the intein was determined. In addition, effects depending on the type of intein using various inteins were determined.

Among various types of inteins, Israel group cysteine-less (iCL) intein, Germany group cysteine-less (gCL) intein, and GP41.1 intein, which do not leave cysteine as a scar during protein trans-splicing, were compared. Meanwhile, Israel group cysteine-less (iCL) intein leaves YIDTDSVYLN as a residual sequence, Germany group cysteine-less (gCL) intein leaves SGDTDS as a residual sequence, and GP41.1 intein leaves SGYSSS as a residual sequence.

### 2-1. Production and purification of botulinum toxin fragments

A plasmid (LC-H_{N}-GP41.1^{N}-R10-H6) was produced by sequentially linking genes encoding the light chain of botulinum toxin (LC, SEQ ID NO: 11), thrombin recognition sequence (SEQ ID NO: 14), a heavy chain translocation domain of botulinum toxin (H_{N}, SEQ ID NO: 12), GP41.1 intein N (GP41.1^{N}, SEQ ID NO: 6), a linker (R10, SEQ ID NO: 4), and a histidine tag (H6 tag, SEQ ID NO: 15) and then transformed into *E. coli* BL21 (DE3).

The transformed *E. coli* was inoculated into 10 ml of terrific broth and then seed-cultured at 37°C and 200 rpm for 16 hours. 8 g of glycerol was added to 1 L of terrific broth to prepare a main culture medium, 10 ml of the seed-cultured solution was inoculated and main culture was performed at 37°C and 200 rpm for 2 hours until the OD₆₀₀ of the culture reached 0.5. IPTG was added to the main culture solution at a concentration of 1 mM and main culture was performed at 16°C and 200 rpm for 16 hours.

The main culture solution was centrifuged at 4°C and 5210 × g for 15 minutes and the supernatant was removed. The pellet obtained by removing the supernatant was resuspended in 150 ml of buffer (50 mM HEPES, 150 mM NaCl, pH 7.4) and the cells were disrupted using an ultrasonic disruptor (10 kHz, 1 sec on/2 sec off, 30 min). 1 ml of the disrupted cells were centrifuged at 19,800 x g to obtain the supernatant, the supernatant was filtered through a 0.22 µm filter, and a botulinum toxin fragment (LC-H_{N}-GP41.1^{N}-R10-H6) containing a light chain (LC) of botulinum toxin and a heavy chain translocation domain (H_{N}) of botulinum toxin was purified.

Meanwhile, the botulinum toxin fragment MBP-R10-GP41.1^{C}-H_{C} (97.7 kDa) and the botulinum toxin fragment LC-HN-GP41.1^{N}-R10-H6 (112.0 kDa) obtained through the process and Example 1 were purified by cation chromatography. The result showed that the two botulinum toxin fragments were produced intact (FIG. 2B).

In addition, botulinum toxin fragments (LC-H_{N}-iCL^{N}-R10-H6, LC-HN-gCL^{N}-R10-H6, MBP-R10-iCL^{C}-H_{C}, MBP-R10-gCL^{C}-H_{C}) were produced and purified using the same process as above and the same method as the process of Example 1 except that the type of intein (iCL^{C}: SEQ ID NO: 7, iCL^{N}: SEQ ID NO: 8, gCL^{C}: SEQ ID NO: 9, gCL^{N}: SEQ ID NO: 10) was changed.

Meanwhile, it can be seen through this process that among Israel group cysteine-less (iCL) intein, Germany group cysteine-less (gCL) intein, and GP41.1 intein, GP41.1 intein expressed a higher amount of botulinum toxin.

### 2-2. Conjugation of botulinum toxin fragments using protein trans-splicing method

TCEP (tris(2-carboxyethyl)phosphine) was added to each of the botulinum toxin fragments (LC-H_{N}-GP41.1^{N}-R10-H6, LC-H_{N}-iCL^{N}-R10-H6, LC-H_{N}-gCL^{N}-R10-H6, MBP-R10-GP41.1^{C}-H_{C}, MBP-R10-iCL^{C}-H_{C}, MBP-R10-gCL^{C}-H_{C}) produced through the process to a concentration of 2 mM and were allowed to stand for 10 minutes. Then, botulinum toxin fragments (LC-H_{N}-GP41.1^{N}-R10-H6, LC-H_{N}-iCL^{N}-R10-H6, LC-HN-gCL^{N}-R10-H6) including the light chain (LC) of botulinum toxin and the heavy chain translocation domain (HN) of botulinum toxin, and botulinum toxin fragments (MBP-R10-GP41.1^{C}-H_{C}, MBP-R10-iCL^{C}-H_{C}, MBP-R10-gCL^{C}-H_{C}) including the heavy-chain receptor-binding domain (HC) of botulinum toxin were mixed at a molar ratio of 1:1 depending on the type of intein, and then were allowed to stand at 20°C and 100 rpm for 2 hours to induce protein trans-splicing (FIGs. 3 and 4).

It can be seen from FIG. 3 that protein trans-splicing completely occurred to produce a recombinant botulinum toxin (LC-H_{N}-H_{C}, rBoNT, 149.5 kDa). On the other hand, it can be seen from FIG. 4 that the protein trans-splicing did not occur when the Israel group cysteine-less (iCL) intein and the Germany group cysteine-less (gCL) intein were used as inteins.

This indicates that, when the botulinum toxin fragments were conjugated using the GP41.1 intein, the intein completely functioned to link the H_{N} and H_{C} of botulinum toxin fragments while removing the histidine tag and the soluble partner, even though the histidine tag and the soluble partner are positioned to interfere with the action of intein.

### 2-3. Production and purification of active botulinum toxin through protease treatment

First, the reducing agent TCEP was removed from the protein trans-splicing reaction product obtained through Example 2-2 by replacing buffer (20 mM Tris, 150 mM NaCl, 2.5 mM CaCl₂, pH 8.0) through dialysis.

Then, the reaction by-product MBP-R10-GP41.1-R10-H6 and the non-reactants LC-H_{N}-GP41.1^{N}-R10-H6, and MBP-R10-GP41.1^{C}-H_{C} could be removed through affinity chromatography (IMAC & MBP) using MBP or H6 at the terminal (FIG. 5B).

Specifically, it can be seen from FIG. 5 that the recombinant botulinum toxin (rBoNT) is not linked to the column and is detected in the unbinding fraction, whereas MBP-R10-GP41.1-R10-H6, LC-H_{N}-GP41.1^{N}-R10-H6, and MBP-R10-GP41.1^{C}-H_{C} are linked to the column and are detected in the eluted fractions.

Meanwhile, the recombinant botulinum toxin (rBoNT) purified from the process was purified again by anion chromatography and then 10 units of thrombin were added thereto and reacted at 20°C for 16 hours to remove the peptide bond between LC and H_{N} of the recombinant botulinum toxin (rBoNT), whereby an active form of recombinant botulinum toxin can be produced (FIG. 6B).

As can be seen from FIG. 6B, the result of SDS-PAGE of the non-reduced (NR) sample that was not treated with a reducing agent shows that rBoNT is detected, whereas the result of the SDS-PAGE of the reduced (R) sample in which the disulfide bond was broken after treated with a reducing agent shows that rBoNT is reduced, and LC and H_{C} are further detected.

It can be seen that the active form of botulinum toxin, in which LC and H_{C} were accurately cleaved by treatment with thrombin, and LC and H_{C} were linked by a disulfide bond, was completely produced. In addition, it can be seen that a dimer of botulinum toxin was not formed by unnecessary disulfide bonds.

### [Sequence List]

### Electronic File is attached hereto

### (C:\KipoNet\NKEditor\appfile\YP-23-160-Specification-GP41-2.xml)

As is apparent from the above description, the present invention shows that Hc having low solubility may be expressed in a soluble form via a linked soluble partner.

The soluble partner is generally a protein with a very large molecular weight and is thus not easy to remove. That is, additional genetic manipulation, such as inserting a restriction enzyme recognition sequence, must be performed to remove the soluble partner. However, in the present invention, it was found that the soluble partner can be easily removed using GP41.1 intein.

In addition, the present invention shows that, among inteins that leave a scar containing no cysteine, GP41.1 intein functions completely and can conjugate H_{N} with H_{C} even though the soluble partner and the purification tag are positioned to interfere with the function of the intein.

In order for botulinum toxin to have complete activity, a disulfide bond mediated by cysteine should be formed between LC and H_{N}. However, when a scar containing cysteine is left, a problem may occur in which a disulfide bond which is different from an original form as described above may be formed, thus causing a problem in the activity of the botulinum toxin. Therefore, it is important to use an intein that leaves a scar that does not contain cysteine. The present invention proves that GP41.1 used herein is an intein that satisfies such a requirement.

In addition, in the present invention, various soluble proteins (MBP, GFP, Sumo) were further fused to the protein to which GP41.1 intein C and the heavy-chain receptor-binding domain (H_{C}) of botulinum toxin were linked. The result shows that the maltose-binding protein (MBP) has the best effect of increasing the solubility expression.

## Claims

1. A method of producing botulinum toxin comprising:
1) expressing and producing each of a botulinum toxin fragment, "LC-H_{N}-GP41.1^{N}", including a light chain (LC) of botulinum toxin, a heavy chain translocation domain (H_{N}) of botulinum toxin, and GP41.1 intein^{N} (GP41.1^{N}) sequentially linked from a 5' end to a 3' end, and a botulinum toxin fragment, "SP-GP41.1^{C}-H_{C}", including a soluble partner (SP) containing polypeptide, GP41.1 intein C(GP41.1^{C}), and a heavy-chain receptor-binding domain (H_{C}) of botulinum toxin sequentially linked from the 5' end to the 3' end;
2) purifying each of the LC-H_{N}-GP41.1^{N} and the SP-GP41.1^{C}-H_{C};
3) removing "GP41.1^{N}" linked to the heavy chain translocation domain (H_{N}) and "SP-GP41.1^{C}" linked to the heavy-chain receptor-binding domain (H_{C}) using a protein trans-splicing method and linking the heavy chain translocation domain (H_{N}) to the heavy-chain receptor-binding domain (H_{C}) without the soluble partner (SP); and
4) cleaving the peptide bond between the light chain (LC) and the heavy chain translocation domain (H_{N}), and inducing a disulfide bond between the cleaved light chain (LC) and heavy chain translocation domain (H_{N}).

2. A method of producing botulinum toxin comprising:
1) expressing and producing each of a botulinum toxin fragment, "LC-H_{N}-GP41.1^{N}", including a light chain (LC) of botulinum toxin, a heavy chain translocation domain (H_{N}) of botulinum toxin, and GP41.1 intein^{N} (GP41.1^{N}) sequentially linked from a 5' end to a 3' end, and a botulinum toxin fragment, "SP-GP41.1^{C}-H_{C}", including a soluble partner (SP) containing polypeptide, GP41.1 intein C(GP41.1^{C}), and a heavy-chain receptor-binding domain (H_{C}) of botulinum toxin sequentially linked from the 5' end to the 3' end;
2) purifying each of the LC-H_{N}-GP41.1^{N} and the SP-GP41.1^{C}-H_{C};
3) cleaving the peptide bond between the light chain (LC) and the heavy chain translocation domain (H_{N}), and inducing a disulfide bond between the cleaved light chain (LC) and heavy chain translocation domain (H_{N}); and
4) removing "GP41.1^{N}" linked to the heavy chain translocation domain (H_{N}) and "SP-GP41.1^{C}" linked to the heavy-chain receptor-binding domain (H_{C}) using a protein trans-splicing method and linking the heavy chain translocation domain (H_{N}) to the heavy-chain receptor-binding domain (H_{C}) without the soluble partner (SP).

3. The method according to claim 1 or 2, wherein the GP41.1 intein N (GP41.1^{N}) is further bound with a purification tag.

4. The method according to claim 1 or 2, wherein the soluble partner is further bound with a purification tag.

5. The method according to claim 1 or 2, wherein the expression occurs in *E. coli.*
